# EUROPEAN PATENT SPECIFICATION

(11) **EP 4 596 027 B1**
(45) Date of publication and mention of the grant of the patent: **04.03.2026**
(21) Application number: 24154792.6
(22) Date of filing: 30.01.2024
(51) Int. Cl.: A61N 1/362, A61N 1/368, A61N 1/37

(54) **METHOD OF DETECTING SUCCESSFUL ENTRAINMENT OF A PART OF A HUMAN HEART**
VERFAHREN ZUM NACHWEIS VON ERFOLGREICHEM MITFÜHREN EINES TEILS EINES MENSCHLICHEN HERZENS
PROCÉDÉ DE DÉTECTION D'ENTRAÎNEMENT RÉUSSIE D'UNE PARTIE D'UN C UR HUMAIN

(43) Date of publication of application: 06.08.2025
(73) Proprietor: CathVision ApS, 2200 Copenhagen (DK)
(72) Inventor: PAAMAD, Rune, 3050 Humlebæk (DK); EL-RASHID, Rami, 2450: København SV (DK); VALVIK, Martin Christian, 34700 Næstved (DK)
(74) Representative: Gottschald Patentanwälte Partnerschaft mbB

(56) References cited:
- EP-B1- 3 174 459
- US-A- 5 855 592
- US-B1- 6 178 351

## Description

The present invention relates to a control system for detecting and displaying successful entrainment of a part of a human heart according to claim 1 and to a respective control unit according to claim 12.

Atrial flutter is a form of cardiac arrhythmia. Often the cause for atrial flutter is an electrical short circuit in the muscle cells of the atrium that allows circular activation patterns. This short circuit can be stopped by ablation therapy if muscle cells that form part of the electrical short circuit are ablated. The circular circuit is then destroyed. To find a good target for ablation therapy, sometimes entrainment of the atrium is performed.

Entrainment is useful if the short circuit (tissue with the circular activations) is activated with a stable rhythm, often with a cycle length of around 200 ms to 250 ms and up to 400 ms. During entrainment, the atrium is paced by stimulations applied through an intracardiac catheter with a stimulation interval slightly below the cycle length of the circular activations (short circuit). For example, if the cycle length of the circular activations is 220 ms, the stimulation interval may be chosen to be 200 ms. If the atrium is successfully entrained, the pacing takes over the activation of the muscle cells that are part of the circular activation. The actual measurement that is then performed is a measurement of the next activation after stopping entrainment. The time difference between the last entrainment stimulation and the first subsequent activation is called post-pacing-interval. The closer the post-pacing-interval is to the non-stimulated cycle length, the closer the electrode measuring the post-pacing-interval is located to the short circuit.

This mechanism is used in practice and provides good results to find ablation targets. However, it has been found that sometimes, a rhythm is non-entrain-able or entrainment is possible but hasn't occurred. Currently, successful entrainment is often visually judged by a physician. If the deviations of the circular activation intervals (cycle length) are in the same order of magnitude as the difference between the stimulation interval and the activation interval, visually confirming successful entrainment, i.e. a change from the activation interval to the stimulation interval is challenging. For example, Miyazaki et. al. in "Entrainment mapping for rapid distinction of left and right atrial tachycardias" Heart Rhythm 2006;3:516 - 523 classify atrial tachycardias as organized and regular if the activation interval has a variability of less than 20 ms. The usual difference between the mean activation interval (cycle length) and the stimulation interval is also 20 ms. It is therefore challenging to visually judge a change in rhythm live during surgery within only a few seconds.

It has been found that misjudgments by physicians of whether entrainment was successful occur. If entrainment was not successful, the measurement of the post-pacing-interval is meaningless and could be misleading. A misjudgment of whether entrainment was successful therefore possibly leads to wrong treatment.

Additionally, long entrainment can induce atrial fibrillation, which is unwanted. The physician therefore should decide quickly whether entrainment was successful and then stop entrainment and measure the post-pacing-interval. That is problematic as entrainment may take some time to occur. Entrainment is therefore a challenging procedure.

Entrainment is also possible in the ventricles (typically with somewhat longer cycle lengths). Possible complications of long entrainments in ventricles are typically that the patient may not tolerate the condition hemodynamically (blood pressure drops) or that they induce ventricular fibrillation (life threatening, requiring cardioversion). The patient may not be hemodynamically stable giving only short opportunities for entrainment.

The known prior art US 2014/0058246 A1 that builds the basis of the invention is related to a method according to claim 1. In this prior art, entrainment is detected by observing a change in the activation interval, i.e. presumably a change from the activation interval to the (entrained) stimulation interval. However, without further information this change can only be detected if the difference between the activation interval and the stimulation interval is relatively large in comparison to the standard deviation of the activation interval. Further, a relatively high number of stimulation intervals needs to be measured to differentiate between changes and deviations in the rhythm of the circular activations and entrainment. Methods to detect entrainment in an implantable pacemaker are known from EP 3 174 459 B1.

Additional methods to detect entrainment are therefore needed.

The invention is based on the problem of improving the known method such that entrainment is detected with higher accuracy.

The above-noted object is solved by the features of claim 1.

The main realization of the present invention is that looking not only at the intracardiac electrogram but also including pacing information allows the detection of entrainment even if deviations of the circular activations and entrainment are in the same order of magnitude or less prominently different than in optimal cases. For example, a statistical analysis of the mean value and standard deviation of the circular activations is possible if it is known that entrainment has not started. Afterwards, if the stimulation interval is known, a statistical analysis of whether the activation intervals during the stimulation have a mean value close to the known stimulation interval can be performed.

Consequently, claim 2 proposes including information about activations prior to the time frame of pacing.

According to claim 3 it is proposed to use the time from stimulation to activation to detect successful entrainment. Before successful entrainment, the time from stimulation to activation will not be constant as the stimulation for example has an interval of 200 ms while the activations have an interval of 220 ms. Over a few activation intervals, an increase in time difference between stimulation and activation is seen if entrainment was not (yet) successful. On the other hand, if entrainment was successful, the time between stimulation and activation should remain relatively constant. The advantage of using this measurement is that it works independently of the actual time between stimulation and activation during entrainment, which may depend on the electrode placement and unknown physiological parameters. If the time between stimulation and activation becomes more or less constant at any value, entrainment was successful. In the medical field, analyzing data in real time may be advantageous, but informing the physician in a detailed manner is also essential. In claim 4 it is therefore proposed to show the time from stimulus to activation to the physician and therefore enable a better understanding of what is happening in the patient and whether entrainment was successful.

Another possible calculation which may be performed additionally or alternatively to detect successful entrainment is whether the activation intervals are statistically close to the stimulation interval (claim 5). The standard deviation of the activation intervals around the stimulation interval for example is a good measurement for this type of "closeness". It is possible to statistically judge the hypothesis that the currently measured activation intervals are not statistically distributed around the stimulation interval.

It is proposed to analyze the success of the entrainment in real time, thereby enabling a physician to make a better decision as to when to stop entrainment. Alternatively, in an after the fact analysis, the reliability of a post-pacing-interval measurement can be determined.

Claim 6 proposes automatically stopping the entrainment and therefore subjecting the patient to less stimulation pulses.

According to embodiments described in claim 7, a morphology template may be derived from activations prior to the pacing, if the start of the pacing is known. It has been found that activations during pacing and before pacing have a different morphology but are still a lot more similar than the activations and pacing artifacts. Pacing artifacts may sometimes be confused with activations and a template is therefore useful to distinguish between artifact, entrained activation and non-entrained activation.

Another teaching according to claim 8, which is of equal importance, relates to a control unit for displaying entrainment information, in particular, the control unit for detecting successful entrainment as proposed and of displaying entrainment information, wherein the control system generates stimulation events by applying electrical energy to stimulation electrodes placed at the part of the human heart to entrain the part of the human heart and generates pacing data relating to the stimulation events, wherein the pacing data comprises a time frame in which the stimulation events occur and a stimulation interval, wherein the control system measures electrical voltages at measurement electrodes placed at the part of the human heart and generates intracardiac electrogram data from the measurements, wherein the intracardiac electrogram data comprises several electrical activations of the part of the human heart in the time frame, wherein the control system comprises a display, wherein the control system displays during the time frame sections of the intracardiac electrogram in a stacked manner.

It has been found that stacking intracardiac electrogram sections makes it visually easy to determine whether entrainment was successful as a non-entrained rhythm has activations which appear to move visually sideways through the sections while an entrained rhythm has activations that appear to stay in one place throughout the stacked sections. This becomes apparent in the different form of displaying the intracardiac electrogram.

All explanations given with regard to the first proposed method are fully applicable.

Claim 9 specifies that the sections are stacked vertically.

It is further advantageous to analyze the sections, in particular their correlation, to detect entrainment (claim 10). Instead of or in addition to visually correlating the sections, they can also be correlated by the control system.

The start of the sections may be manually moved by a user, for example in the case where activations are building up between two sections and therefore difficult to see. The length will remain the same.

Another teaching according to claim 12, which is of equal importance, relates to a control unit for detecting successful entrainment of a part of a human heart, in particular of an atrium, wherein the control unit receives or generates pacing data, wherein the pacing data comprises a time frame in which the stimulation events occur, wherein the control unit receives or generates intracardiac electrogram data, wherein the intracardiac electrogram data comprises several electrical activations of the part of the human heart in the time frame, wherein the control unit detects at least some of the electrical activations in the time frame, wherein the control unit derives one or more parameters of the stimulation events from the pacing data, wherein the control unit derives one or more parameters of the electrical activations in the time frame, wherein the control unit derives from the one or more parameters of the stimulation events and the one or more parameters of the electrical activations in the time frame whether the part of the human heart was successfully entrained in the time frame.

The control unit performs some of the functions of the control system. In particular, measuring the intracardiac electrogram and providing stimulations for pacing may be done by other control units, possibly of other manufacturers. Together, the control units may form the control system.

All explanations given with regard to the proposed methods are fully applicable.

In the following, embodiments of the invention are explained with respect to the drawing. The drawing shows in
- Fig. 1,: schematically a heart with catheters, electrodes on the catheters and a control system,
- Fig. 2,: an intracardiac electrogram with an additional channel showing pacing stimuli,
- Fig. 3,: time differences between stimuli and activations and
- Fig. 4,: stacked sections of channels.

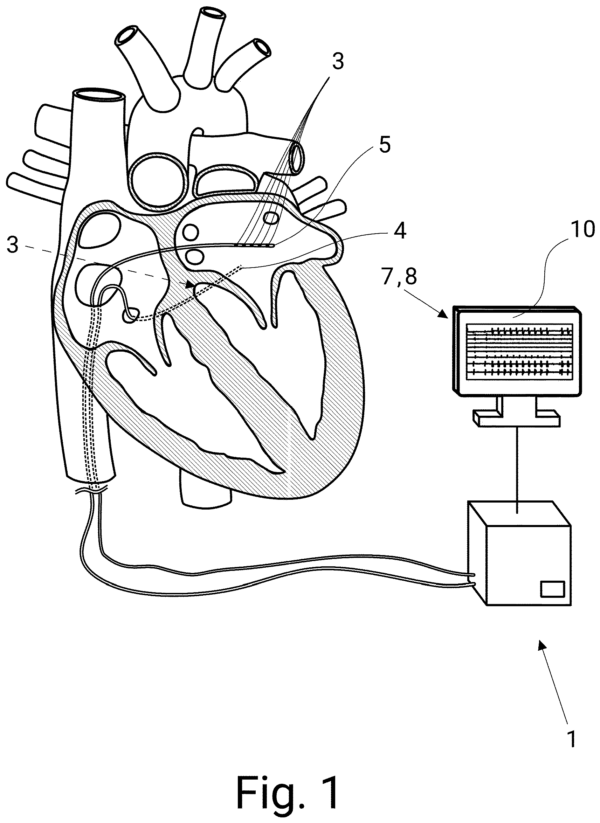

Proposed, however not claimed, is a method of detecting successful entrainment of a part of a human heart, in particular of an atrium, wherein the method is implemented by a control system 1. Fig. 1 shows a control system 1 and schematically a surgery situation in which the control system 1 may be used. According to the proposed method, the control system 1 detects whether entrainment has been successful according to a predefined criterion. The method may be performed with regard to a patient with atrial flutter during an atrial flutter episode.

The control system 1 generates stimulation events 2 by applying electrical energy to stimulation electrodes 3 placed at the part of the human heart to entrain the part of the human heart. Fig. 1 shows a coronary sinus catheter 4 and an ablation catheter 5, each with electrodes 3. Other catheters may be present. The control system 1 generates pacing data relating to the stimulation events 2. The pacing data comprises a time frame 6 in which the stimulation events 2 occur. The time frame 6 begins when the pacing, i.e. entrainment, starts. It ends, when the pacing ends. The time frame 6 may be ongoing during the execution of the proposed method.

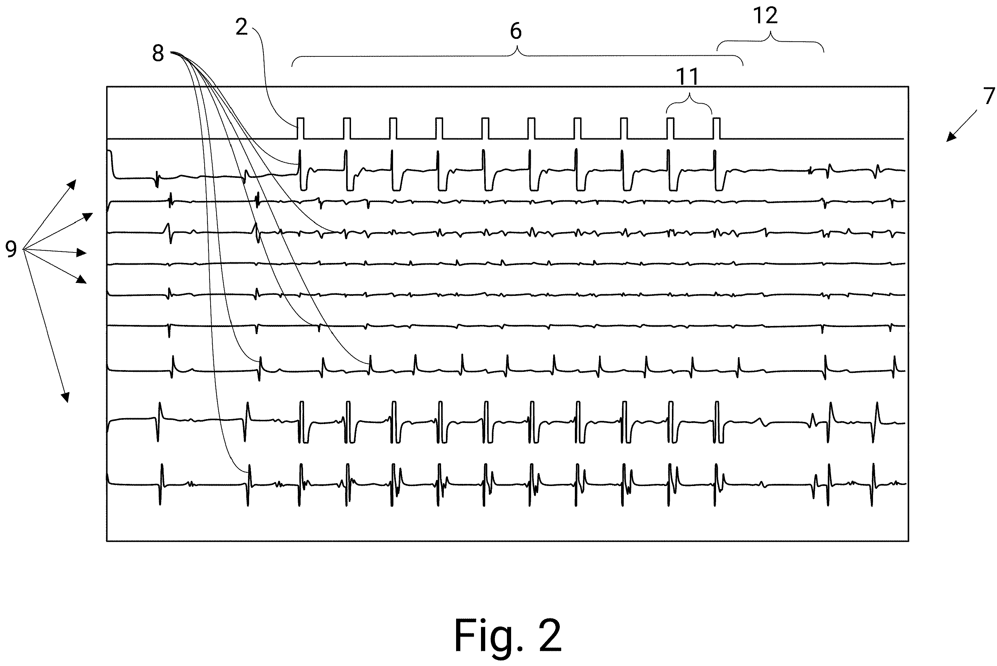

The control system 1 measures electrical voltages at measurement electrodes 3 placed at the part of the human heart and generates intracardiac electrogram data 7 from the measurements. It is presently not relevant which electrodes 3 are used for pacing and which for measurement or whether the same electrodes 3 are used. The intracardiac electrogram data 7 comprises several electrical activations 8 of the part of the human heart in the time frame 6. Here and preferably, the intracardiac electrogram comprises several channels 9 with several electrical activations 8 each. The channels 9 may comprise bipolar and/or unipolar channels 9. Fig. 2 shows several channels 9 of the intracardiac electrogram. From the topmost channel 9, it can be seen when the pacing starts. This topmost channel 9 is not an intracardiac electrogram channel 9 but only shows the pacing stimuli here. At least the second topmost channel 9 and the second channel 9 from the bottom show strong pacing artifacts. In reality, those are often difficult to distinguish from real activations and/or overlap with real activations, making it hard to identify whether entrainment was successful. In Fig. 2, the channels 9 are idealized and therefore easier to read.

The control system 1 may comprise a processor and/or a memory. The control system 1 may be a local unit with a processor, possibly a user interface and the like. It may also comprise in one embodiment a cloud processor. It is therefore not necessary for the control system 1 to be confined to a single device. The control system 1 may comprise an interface for one or more catheters. The one or more catheters may comprise the measurement electrodes 3 and the stimulation electrodes 3. It is conceivable that the stimulation electrodes 3 and the measurement electrodes 3 are the same electrodes 3. The control system 1 is preferably configured to perform the proposed method. The control system 1 may therefore comprise computer code, in particular saved in the memory, that, when executed, performs the steps of the method. The control system 1 comprises a display 10. Fig. 2 to 4 show graphical representations that may be displayed on the display 10, in particular in different areas of the display 10 or by selecting different menu points.

The control system 1 detects at least some of the electrical activations 8 in the time frame 6. Algorithms to detect activations are known, preferred details of algorithms will be described below.

The control system 1 derives one or more parameters of the stimulation events 2 from the pacing data. In particular, the one or more parameters of the stimulation events 2 may comprise a stimulation interval 11. Usually, this stimulation interval 11 is input by a user such that the control system 1 does not need to measure the stimulation interval 11 and can instead read it from memory and use it for pacing.

The control system 1 derives one or more parameters of the electrical activations 8 in the time frame 6, in particular an activation interval. The activation interval prior to the time frame 6 is usually around 20 to 30 ms higher than the stimulation interval 11. If entrainment was successful, the activation interval in the time frame 6 should be close to the stimulation interval 11.

Therefore, the control system 1 derives from the one or more parameters of the stimulation events 2 and the one or more parameters of the electrical activations 8 in the time frame 6 whether the part of the human heart was successfully entrained in the time frame 6.

For detecting the success of entrainment in one embodiment the pacing data comprises a time of a beginning of the time frame 6 and the intracardiac electrogram data 7 comprises several electrical activations 8 of the part of the human heart prior to the time frame 6. The control system 1 may detect at least some of the electrical activations 8 prior to the time frame 6 and derive one or more parameters of the electrical activations 8 prior to the time frame 6. Preferably, the one or more parameters of the electrical activations 8 prior to the time frame 6 comprise activation intervals. It should be understood that the term "interval" herein relates to the length of the respective interval or to several lengths of the respective interval, i.e. the length distribution.

It is proposed in one embodiment that the one or more parameters of the stimulation events 2 comprise a time of the stimulation events 2. The time may be the time of a beginning or a peak of the stimulation or the like. Preferably, only a single time basis is used such that the times of different events are well comparable.

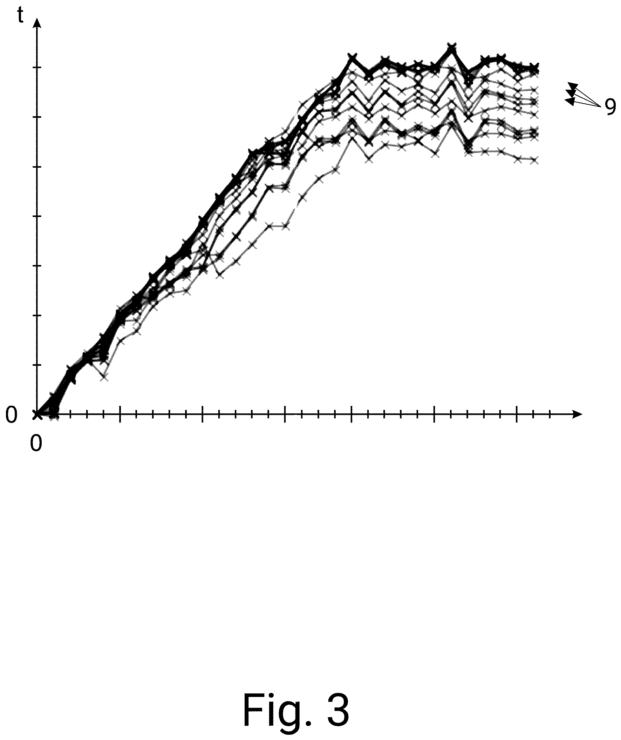

The one or more parameters of the electrical activations 8 in the time frame 6 may comprise a time of the electrical activations 8. The control system 1 derives time differences between the stimulation events 2 and subsequent electrical activations 8 in the time frame 6 for several stimulation events 2 to derive whether entrainment was successful in the time frame 6. Fig. 3 shows on the y-axis the time difference between stimulation and activation and on the x-axis the stimulations (i.e. first, stimulation, second stimulation, ...). It is clearly apparent at what point the time differences start being constant. At that point, the rhythm was entrained. Previously, due to the differences between activation interval and stimulation interval 11, the rhythm was not entrained and the time differences increase with each activation. For example, in a non-entrained rhythm, if the first stimulation and activation are at 0 ms, the subsequent stimulation would be at e.g. 200 ms and the subsequent activation at 220 ms, followed by 400 ms; 440 ms, then 600 ms; 660 ms and so on. At some point, if the rhythm is entrained, the activations start having an interval that is also 200 ms and the time difference stays constant. The graphical representation in Fig. 3 is one way of making entrainment visible for the human eye compared to watching a running intracardiac electrogram as in Fig. 2.

Preferably, the control system 1 derives a statistical parameter, in particular a standard deviation, from the time differences and if the statistical parameter meets a predefined criterion, concludes that entrainment was successful. A standard deviation of. for example, three consecutive activations would be very different during entrainment and non-entrainment. Other statistical parameters may be derived from stochastic modeling such as Markov models or Bayesian methods where a probability estimate of successful entrainment is preferably updated with every new observation of activations 8.

The predefined criterion may be based on the one or more parameters of the stimulation events 2 and/or on the one or more parameters of the activation events prior to the time frame 6. For example, the standard deviation here and preferably needs to be below 10 % of the activation interval prior to the time frame 6 or of the stimulation interval 11. Here and preferably, the control system 1 analyzes between 3 and 10, preferably between 3 and 6 consecutive activations in real time to detect entrainment quickly. The term "real time" should be understood to possibly include significant delays, but still be close enough to reality, as generally used in the art.

The control system 1 may display the time differences between the stimulation events 2 and subsequent electrical activations 8, for example as shown in Fig. 3.

Another embodiment for detecting entrainment may be used alternatively or in addition, for example in a combined algorithm, and is now described. The one or more parameters of the stimulation events 2 may comprise a stimulation interval 11. The one or more parameters of the electrical activations 8 in the time frame 6 may comprise activation intervals, which preferably are extracted by the control system 1. The control system 1 may compare the stimulation interval 11 to several activation intervals of the electrical activations 8 in the time frame 6 to derive whether entrainment was successful.

Preferably, the control system 1 derives a statistical parameter, in particular a standard deviation, from the several activation intervals of the electrical activations 8 in the time frame 6 in relation to the stimulation interval 11 and if the statistical parameter meets a predefined criterion, concludes that entrainment was successful. Preferably, the predefined criterion is based on the one or more parameters of the stimulation events 2. The control system 1 may use the hypothesis that the mean value of the activation intervals is the stimulation interval 11 and calculate the standard deviation based on that assumption. If the activation interval is close to the stimulation interval 11, the standard deviation will be low. Again, for example, the standard deviation here and preferably needs to be below 10 % of the activation interval prior to the time frame 6 or of the stimulation interval 11. Again, here and preferably, the control system 1 analyzes between 3 and 10, preferably between 3 and 6 consecutive activations in real time to detect entrainment quickly.

It is proposed, that the control system 1 determines whether entrainment was successful in real time during the time frame 6. The control system 1 sends the determination whether entrainment was successful to a display 10 of the control system 1 for displaying the determination to a user. The control system 1 may receive and/or measure the intracardiac electrogram continuously.

The control system 1 after determining that entrainment was successful stops generating stimulation events 2. Pacing and the time frame 6 then end. Alternatively, a user may input a stop signal and end the pacing. In both cases the intracardiac electrogram data 7 may comprise at least one electrical activation 8 of the part of the human heart after the time frame 6 and the control system 1 after determining that entrainment was successful may detect the first electrical activation 8 after the time frame 6 and derive a post-pacing-interval 12 from the first electrical activation 8 after the time frame 6 and a last electrical activation 8 in the time frame 6. The post-pacing-interval 12 may be defined as the interval between the last activation in the time frame 6 and the next activation or as the time frame 6 between the last stimulation and the next activation.

According to one embodiment it is proposed that the control system 1 derives a morphology template prior to the time frame 6 from at least some of the electrical activations 8 prior to the time frame 6. The morphology templates may be derived with any known method.

The control system 1 may use the morphology template to detect electrical activations 8 in the time frame 6 and/or to differentiate between electrical activations 8 and pacing artifacts in the time frame 6 and/or to determine whether entrainment was successful and/or to display and/or highlight a channel 9 of the intracardiac electrogram. In the time frame 6 and prior to the time frame 6, the activations, at least during entrainment, are noticeably different, but much closer to each other in terms of morphology than to pacing artifacts. That can be used to differentiate between the three and to detect entrainment. It is also possible to highlight or display a channel 9 with high correlation values as probably optically well usable to visually detect entrainment. As Fig. 2 shows, some channels 9 make seeing activations a lot more difficult than others and further some channels 9 have pacing artifacts.

In another embodiment, the detection of electrical activations 8 by the control system 1 is performed on the bipolar and unipolar channels 9 of an electrode 3, preferably by detecting a window for an activation in the bipolar channel 9 and then detecting the peak or another time information in the unipolar channel 9.

Another teaching, which however is not claimed, relates to a method of displaying entrainment information, in particular method of detecting successful entrainment as described previously and of displaying entrainment information. All explanations given with regard to each method may be applied to the other method.

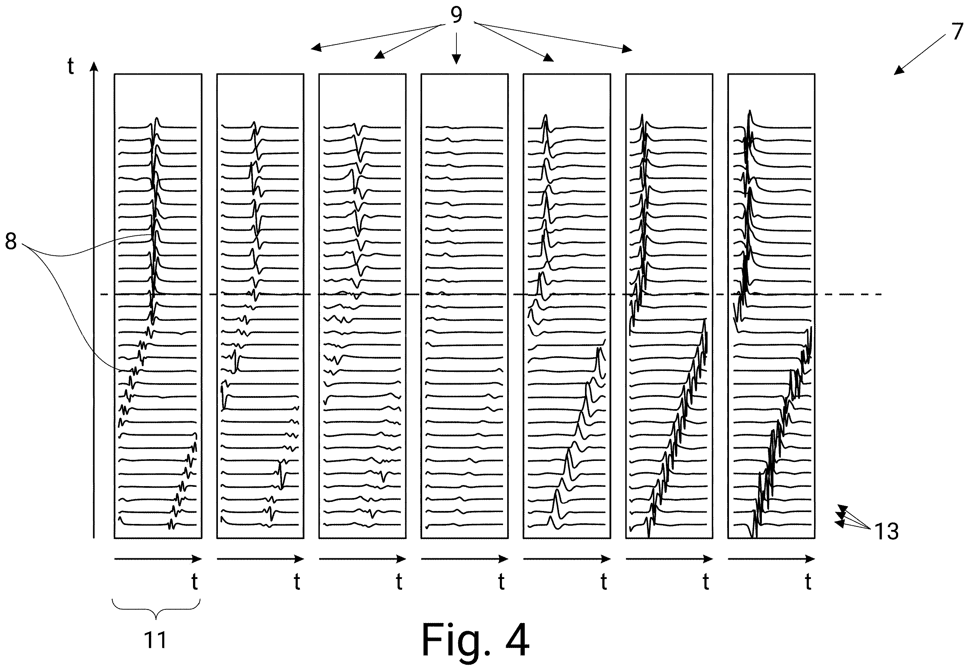

The method is implemented by a control system 1, here and preferably the same control system 1 as before. The control system 1 generates stimulation events 2 by applying electrical energy to stimulation electrodes 3 placed at the part of the human heart to entrain the part of the human heart and generates pacing data relating to the stimulation events 2. The pacing data comprises a time frame 6 in which the stimulation events 2 occur and a stimulation interval 11. The control system 1 measures electrical voltages at measurement electrodes 3 placed at the part of the human heart and generates intracardiac electrogram data 7 from the measurements. The intracardiac electrogram data 7 comprises several electrical activations 8 of the part of the human heart in the time frame 6. The control system 1 comprises a display 10. The control system 1 displays during the time frame 6 sections 13 of the intracardiac electrogram, preferably with a length of a natural number multiple of the stimulation interval 11, in particular with a length of the stimulation interval 11, in a stacked manner. The stacked sections 13 are here and preferably aligned to the stimulation time or the activation time, here to the stimulation time. Each section 13 in Fig. 4 starts with a new stimulation time, but may also start at a constant time after a stimulation time, possibly with the constant time being configurable as will be described. The stimulation time and the activation time are the time of the start or any other feature of the stimulation or activation respectively. Preferably, the sections don't overlap, but that is not strictly necessary.

Fig. 4 shows an embodiment of stacked sections 13. Noticeably, in Fig. 4, the time dimension of the electrogram is split into sections 13 going from left to right and into the stacking of the sections 13 from bottom to top. This visualization makes it easier to spot entrainment. In the bottom half of Fig. 4, entrainment has not happened. The activations seem to be running sideways in the sections 13 when read from bottom to top as the activation interval is greater than the stimulation interval 11 and the sections 13 have the lengths on one stimulation interval 11. It is apparent that in the top half, entrainment was successful as the activations stop moving sideways through the sections 13. Compared to Fig. 2, visually identifying that the activation interval and the stimulation interval 11 are more or less the same has become a lot easier for most channels 9.

It is here the case that the control system 1 displays a time from stimulus to electrical activation 8 for several electrical activations 8 during the time frame 6 independently of a detection of the electrical activation 8 or without detecting the electrical activation 8. For this method of displaying it is not necessary to correctly detect activations, completely removing any possible mistakes in detection from the entrainment assessment. The stimulus interval 11 is known perfectly well and the control system 1 only cuts the channels 9 into sections 13.

According to one embodiment it is proposed that the control system 1 displays the sections 13 such that subsequent sections 13 are displayed above or below each other while the time dimension of each section 13 is oriented sideways.

It may further be the case that the control system 1 determines a correlation between at least some of the sections 13 and derives a quality indicator, in particular for the determination of the success of the entrainment, from the correlation. As is apparent from Fig. 4, if subsequent sections 13 correlate strongly, entrainment is likely.

Here and preferably, the user may move the start of the sections 13 via a user input. In Fig. 4, the activations stack more or less in the middle of the sections 13. However, it is possible for them to stack right at the edge of the sections 13. In that case, moving the start of all sections 13 is sensible.

Another teaching which is of equal importance relates to a control unit for detecting successful entrainment of a part of a human heart, in particular of an atrium, wherein the control unit receives or generates pacing data, wherein the pacing data comprises a time frame 6 in which the stimulation events 2 occur, wherein the control unit receives or generates intracardiac electrogram data 7, wherein the intracardiac electrogram data 7 comprises several electrical activations 8 of the part of the human heart in the time frame 6, wherein the control unit detects at least some of the electrical activations 8 in the time frame 6, wherein the control unit derives one or more parameters of the stimulation events 2 from the pacing data, wherein the control unit derives one or more parameters of the electrical activations 8 in the time frame 6, wherein the control unit derives from the one or more parameters of the stimulation events 2 and the one or more parameters of the electrical activations 8 in the time frame 6 whether the part of the human heart was successfully entrained in the time frame 6.

The control unit is part of the described control system 1. Any described function of the control system 1 may be performed by the control unit. The control unit may be connectable to other control units for pacing and measurement. The control unit may comprise the described display 10.

Another teaching which is of equal importance relates to a control system 1 configured for use in the proposed method.

## Claims

1. Control system for detecting successful entrainment of a part of a human heart, in particular of an atrium,
wherein the control system (1) generates stimulation events (2) by applying electrical energy to stimulation electrodes (3) placed at the part of the human heart to entrain the part of the human heart and generates pacing data relating to the stimulation events (2), wherein the pacing data comprises a time frame (6) in which the stimulation events (2) occur,
wherein the control system (1) measures electrical voltages at measurement electrodes (3) placed at the part of the human heart and generates intracardiac electrogram data (7) from the measurements, wherein the intracardiac electrogram data (7) comprises several electrical activations (8) of the part of the human heart in the time frame (6),
wherein the control system (1) detects at least some of the electrical activations (8) in the time frame (6),
wherein the control system (1) derives one or more parameters of the stimulation events (2) from the pacing data, wherein the control system (1) derives one or more parameters of the electrical activations (8) in the time frame (6),
wherein the control system (1) derives from the one or more parameters of the stimulation events (2) and the one or more parameters of the electrical activations (8) in the time frame (6) whether the part of the human heart was successfully entrained in the time frame (6),
wherein the control system (1) comprises a display (10),
wherein the control system (1) determines whether entrainment was successful in real time during the time frame (6), wherein the control system (1) sends the determination whether entrainment was successful to the display (10) of the control system (1) for displaying the determination to a user,
wherein the control system (1) after determining that entrainment was successful stops generating stimulation events (2), or, wherein a user may input a stop signal and end the pacing.

2. Control system according to claim 1, **characterized in that** the pacing data comprises a time of a beginning of the time frame (6), that the intracardiac electrogram data (7) comprises several electrical activations (8) of the part of the human heart prior to the time frame (6), that the control system (1) detects at least some of the electrical activations (8) prior to the time frame (6), that the control system (1) derives one or more parameters of the electrical activations (8) prior to the time frame (6), preferably, that the one or more parameters of the electrical activations (8) prior to the time frame (6) comprise activation intervals.

3. Control system according to claim 1 or 2, **characterized in that** the one or more parameters of the stimulation events (2) comprise a time of the stimulation events (2), that the one or more parameters of the electrical activations (8) in the time frame (6) comprise a time of the electrical activations (8), that the control system (1) derives time differences between the stimulation events (2) and subsequent electrical activations (8) in the time frame (6) for several stimulation events (2) to derive whether entrainment was successful in the time frame (6), preferably, that the control system (1) derives a statistical parameter, in particular a standard deviation, from the time differences and if the statistical parameter meets a predefined criterion, concludes that entrainment was successful, more preferably, that the predefined criterion is based on the one or more parameters of the stimulation events (2) and/or on the one or more parameters of the activation events prior to the time frame (6).

4. Control system according to one of the preceding claims, **characterized in that** the control system (1) displays the time differences between the stimulation events (2) and subsequent electrical activations (8).

5. Control system according to one of the preceding claims, **characterized in that** the one or more parameters of the stimulation events (2) comprise a stimulation interval (11), that the one or more parameters of the electrical activations (8) in the time frame (6) comprise activation intervals, that the control system (1) compares the stimulation interval (11) to several activation intervals of the electrical activations (8) in the time frame (6) to derive whether entrainment was successful, preferably, that the control system (1) derives a statistical parameter, in particular a standard deviation, from the several activation intervals of the electrical activations (8) in the time frame (6) in relation to the stimulation interval (11) and if the statistical parameter meets a predefined criterion, concludes that entrainment was successful, more preferably, that the predefined criterion is based on the one or more parameters of the stimulation events (2).

6. Control system according to one of the preceding claims, **characterized in that** the intracardiac electrogram data (7) comprises at least one electrical activation (8) of the part of the human heart after the time frame (6), that the control system (1) after determining that entrainment was successful detects the first electrical activation (8) after the time frame (6) and derives a post-pacing-interval (12) from the first electrical activation (8) after the time frame (6) and a last electrical activation (8) in the time frame (6).

7. Control system according to one of claims 2 to 6, **characterized in that** the control system (1) derives a morphology template prior to the time frame (6) from at least some of the electrical activations (8) prior to the time frame (6), and, that the control system (1) uses the morphology template to detect electrical activations (8) in the time frame (6) and/or to differentiate between electrical activations (8) and pacing artifacts in the time frame (6) and/or to determine whether entrainment was successful and/or to display and/or highlight a channel (9) of the intracardiac electrogram.

8. Control system for detecting successful entrainment according to one of the previous claims and of displaying entrainment information,
wherein the control system (1) generates stimulation events (2) by applying electrical energy to stimulation electrodes (3) placed at the part of the human heart to entrain the part of the human heart and generates pacing data relating to the stimulation events (2), wherein the pacing data comprises a time frame (6) in which the stimulation events (2) occur and a stimulation interval (11),
wherein the control system (1) measures electrical voltages at measurement electrodes (3) placed at the part of the human heart and generates intracardiac electrogram data (7) from the measurements, wherein the intracardiac electrogram data (7) comprises several electrical activations (8) of the part of the human heart in the time frame (6),
wherein the control system (1) comprises a display (10),
wherein the control system (1) displays during the time frame (6) sections (13) of the intracardiac electrogram in a stacked manner.

9. Control system according to claim 8, **characterized in that** the control system (1) displays the sections (13) such that subsequent sections (13) are displayed above or below each other while the time dimension of each section (13) is oriented sideways.

10. Control system according to claim 8 or 9, **characterized in that** the control system (1) determines a correlation between at least some of the sections (13) and derives a quality indicator, in particular for the determination of the success of the entrainment, from the correlation.

11. Control system according to one of the claims 8 to 10, **characterized in that** the user may move a start of the sections (13) via a user input.

12. Control unit for detecting successful entrainment of a part of a human heart, in particular of an atrium, adapted to be part of a control system (1) according to one of the preceding claims, wherein the control unit receives pacing data, wherein the pacing data comprises a time frame (6) in which the stimulation events (2) occur,
wherein the control unit receives or generates intracardiac electrogram data (7), wherein the intracardiac electrogram data (7) comprises several electrical activations (8) of the part of the human heart in the time frame (6),
wherein the control unit detects at least some of the electrical activations (8) in the time frame (6),
wherein the control unit derives one or more parameters of the stimulation events (2) from the pacing data, wherein the control unit derives one or more parameters of the electrical activations (8) in the time frame (6),
wherein the control unit derives from the one or more parameters of the stimulation events (2) and the one or more parameters of the electrical activations (8) in the time frame (6) whether the part of the human heart was successfully entrained in the time frame (6),
wherein the control system (1) comprises a display (10),
wherein the control unit determines whether entrainment was successful in real time during the time frame (6), wherein the control unit sends the determination whether entrainment was successful to the display (10) of the control system (1) for displaying the determination to a user.

## Patentansprüche

1. Steuersystem zum Erkennen einer erfolgreichen Synchronisation (Entrainment) eines Teils eines menschlichen Herzens, insbesondere eines Vorhofs auf einen Stimulus,
wobei das Steuersystem (1) Stimulationsereignisse (2) erzeugt, indem es elektrische Energie an Stimulationselektroden (3) anlegt, die an dem Teil des menschlichen Herzens angeordnet sind, um den Teil des menschlichen Herzens auf den Stimulus zu synchronisieren, und Stimulationsdaten erzeugt, die sich auf die Stimulationsereignisse (2) beziehen, wobei die Stimulationsdaten einen Zeitrahmen (6) umfassen, in dem die Stimulationsereignisse (2) auftreten,
wobei das Steuersystem (1) elektrische Spannungen an Messelektroden (3) misst, die an dem Teil des menschlichen Herzens platziert sind, und aus den Messungen intrakardiale Elektrogrammdaten (7) erzeugt, wobei die intrakardialen Elektrogrammdaten (7) mehrere elektrische Aktivierungen (8) des Teils des menschlichen Herzens innerhalb des Zeitrahmens (6) umfassen,
wobei das Steuersystem (1) zumindest einige der elektrischen Aktivierungen (8) im Zeitrahmen (6) erkennt, wobei das Steuersystem (1) aus den Stimulationsdaten einen oder mehrere Parameter der Stimulationsereignisse (2) ableitet, wobei das Steuersystem (1) einen oder mehrere Parameter der elektrischen Aktivierungen (8) im Zeitrahmen (6) ableitet, wobei das Steuersystem (1) aus den ein oder mehreren Parametern der Stimulationsereignisse (2) und den ein oder mehreren Parametern der elektrischen Aktivierungen (8) im Zeitrahmen (6) ableitet, ob der Teil des menschlichen Herzens im Zeitrahmen (6) erfolgreich auf den Stimulus synchronisiert wurde,
wobei das Steuersystem (1) eine Anzeige (10) umfasst,
wobei das Steuersystem (1) bestimmt, ob die Synchronisation in Echtzeit während des Zeitrahmens (6) erfolgreich war, wobei das Steuersystem (1) die Bestimmung, ob die Synchronisation erfolgreich war, an die Anzeige (10) des Steuersystems (1) sendet, um die Bestimmung einem Benutzer anzuzeigen,
wobei das Steuersystem (1) nach dem Bestimmen, dass die Synchronisation erfolgreich war, das Erzeugen von Stimulationsereignissen (2) beendet, oder wobei ein Benutzer ein Stoppsignal eingeben und die Stimulation beenden kann.

2. Steuersystem nach Anspruch 1, **dadurch gekennzeichnet, dass** die Stimulationsdaten einen Zeitpunkt eines Beginns des Zeitrahmens (6) umfassen, dass die intrakardialen Elektrogrammdaten (7) mehrere elektrische Aktivierungen (8) des Teils des menschlichen Herzens vor dem Zeitrahmen (6) umfassen, dass das Steuersystem (1) mindestens einige der elektrischen Aktivierungen (8) vor dem Zeitrahmen (6) erkennt, dass das Steuersystem (1) einen oder mehrere Parameter der elektrischen Aktivierungen (8) vor dem Zeitrahmen (6) ableitet, vorzugsweise, dass die ein oder mehreren Parameter der elektrischen Aktivierungen (8) vor dem Zeitrahmen (6) Aktivierungsintervalle umfassen.

3. Steuersystem nach Anspruch 1 oder 2, **dadurch gekennzeichnet, dass** die ein oder mehreren Parameter der Stimulationsereignisse (2) einen Zeitpunkt der Stimulationsereignisse (2) umfassen, dass die ein oder mehreren Parameter der elektrischen Aktivierungen (8) im Zeitrahmen (6) einen Zeitpunkt der elektrischen Aktivierungen (8) umfassen, dass das Steuersystem (1) Zeitunterschiede zwischen den Stimulationsereignissen (2) und nachfolgenden elektrischen Aktivierungen (8) im Zeitrahmen (6) für mehrere Stimulationsereignisse (2) ableitet, um abzuleiten, ob die Synchronisation im Zeitrahmen (6) erfolgreich war, vorzugsweise, dass das Steuersystem (1) aus den Zeitdifferenzen einen statistischen Parameter, insbesondere eine Standardabweichung, ableitet und, wenn der statistische Parameter ein vordefiniertes Kriterium erfüllt, zu dem Schluss kommt, dass die Synchronisation erfolgreich war, noch bevorzugter, dass das vordefinierte Kriterium auf den ein oder mehreren Parametern der Stimulationsereignisse (2) und/oder auf den ein oder mehreren Parametern der Aktivierungsereignisse vor dem Zeitrahmen (6) basiert.

4. Steuersystem nach einem der vorstehenden Ansprüche, **dadurch gekennzeichnet, dass** das Steuersystem (1) die Zeitdifferenzen zwischen den Stimulationsereignissen (2) und nachfolgenden elektrischen Aktivierungen (8) anzeigt.

5. Steuersystem nach einem der vorstehenden Ansprüche, **dadurch gekennzeichnet, dass** die ein oder mehreren Parameter der Stimulationsereignisse (2) ein Stimulationsintervall (11) umfassen, dass die ein oder mehreren Parameter der elektrischen Aktivierungen (8) im Zeitrahmen (6) Aktivierungsintervalle umfassen, dass das Steuersystem (1) das Stimulationsintervall (11) mit mehreren Aktivierungsintervallen der elektrischen Aktivierungen (8) im Zeitrahmen (6) vergleicht, um abzuleiten, ob die Synchronisation erfolgreich war, vorzugsweise, dass das Steuersystem (1) einen statistischen Parameter, insbesondere eine Standardabweichung, aus den mehreren Aktivierungsintervallen der elektrischen Aktivierungen (8) im Zeitrahmen (6) in Bezug auf das Stimulationsintervall (11) ableitet und, wenn der statistische Parameter ein vordefiniertes Kriterium erfüllt, zu dem Schluss kommt, dass die Synchronisation erfolgreich war, noch bevorzugter, dass das vordefinierte Kriterium auf den ein oder mehreren Parametern der Stimulationsereignisse (2) basiert.

6. Steuersystem nach einem der vorstehenden Ansprüche, **dadurch gekennzeichnet, dass** die intrakardialen Elektrogrammdaten (7) mindestens eine elektrische Aktivierung (8) des Teils des menschlichen Herzens nach dem Zeitrahmen (6) umfassen, dass das Steuersystem (1) nach dem Bestimmen, dass die Synchronisation erfolgreich war, die erste elektrische Aktivierung (8) nach dem Zeitrahmen (6) erkennt und ein Nachstimulationsintervall (12) aus der ersten elektrischen Aktivierung (8) nach dem Zeitrahmen (6) und einer letzten elektrischen Aktivierung (8) im Zeitrahmen (6) ableitet.

7. Steuersystem nach einem der Ansprüche 2 bis 6, **dadurch gekennzeichnet, dass** das Steuersystem (1) vor dem Zeitrahmen (6) eine Morphologievorlage aus zumindest einigen der elektrischen Aktivierungen (8) vor dem Zeitrahmen (6) ableitet und dass das Steuersystem (1) die Morphologievorlage verwendet, um elektrische Aktivierungen (8) im Zeitrahmen (6) zu erkennen und/oder um zwischen elektrischen Aktivierungen (8) und Stimulationsartefakten im Zeitrahmen (6) zu unterscheiden und/oder um zu bestimmen, ob die Synchronisation erfolgreich war, und/oder um einen Kanal (9) des intrakardialen Elektrogramms anzuzeigen und/oder hervorzuheben.

8. Steuersystem zum Erkennen einer erfolgreichen Synchronisation gemäß einem der vorherigen Ansprüche und zum Anzeigen von Synchronisationsinformationen,
wobei das Steuersystem (1) Stimulationsereignisse (2) erzeugt, indem es elektrische Energie an Stimulationselektroden (3) anlegt, die an dem Teil des menschlichen Herzens angeordnet sind, um den Teil des menschlichen Herzens auf einen Stimulus zu synchronisieren, und Stimulationsdaten erzeugt, die sich auf die Stimulationsereignisse (2) beziehen, wobei die Stimulationsdaten einen Zeitrahmen (6), in dem die Stimulationsereignisse (2) auftreten, und ein Stimulationsintervall (11) umfassen, wobei das Steuersystem (1) elektrische Spannungen an Messelektroden (3) misst, die an dem Teil des menschlichen Herzens angeordnet sind, und aus den Messungen intrakardiale Elektrogrammdaten (7) erzeugt, wobei die intrakardialen Elektrogrammdaten (7) mehrere elektrische Aktivierungen (8) des Teils des menschlichen Herzens im Zeitrahmen (6) umfassen,
wobei das Steuersystem (1) eine Anzeige (10) umfasst, wobei das Steuersystem (1) während des Zeitrahmens (6) Abschnitte (13) des intrakardialen Elektrogramms gestapelt anzeigt.

9. Steuersystem nach Anspruch 8, **dadurch gekennzeichnet, dass** das Steuersystem (1) die Abschnitte (13) so anzeigt, dass aufeinanderfolgende Abschnitte (13) über- oder untereinander angezeigt werden, während die zeitliche Dimension jedes Abschnitts (13) seitlich ausgerichtet ist.

10. Steuersystem nach Anspruch 8 oder 9, **dadurch gekennzeichnet, dass** das Steuersystem (1) eine Korrelation zwischen zumindest einigen der Abschnitte (13) bestimmt und einen Qualitätsindikator, insbesondere zur Bestimmung des Erfolgs der Synchronisation, aus der Korrelation ableitet.

11. Steuersystem nach einem der Ansprüche 8 bis 10, **dadurch gekennzeichnet, dass** der Benutzer einen Anfang der Abschnitte (13) über eine Benutzereingabe verschieben kann.

12. Steuereinheit zum Erkennen einer erfolgreichen Synchronisation (Entrainment) eines Teils eines menschlichen Herzens, insbesondere eines Vorhofs, auf einen Stimulus, die dazu ausgelegt ist, Teil eines Steuersystems (1) gemäß einem der vorstehenden Ansprüche zu sein, wobei die Steuereinheit Stimulationsdaten empfängt, wobei die Stimulationsdaten einen Zeitrahmen (6) umfassen, in dem die Stimulationsereignisse (2) auftreten,
wobei die Steuereinheit intrakardiale Elektrogrammdaten (7) empfängt oder erzeugt, wobei die intrakardialen Elektrogrammdaten (7) mehrere elektrische Aktivierungen (8) des Teils des menschlichen Herzens innerhalb des Zeitrahmens (6) umfassen,
wobei die Steuereinheit zumindest einige der elektrischen Aktivierungen (8) im Zeitrahmen (6) erkennt,
wobei die Steuereinheit einen oder mehrere Parameter der Stimulationsereignisse (2) aus den Stimulationsdaten ableitet, wobei die Steuereinheit einen oder mehrere Parameter der elektrischen Aktivierungen (8) im Zeitrahmen (6) ableitet,
wobei die Steuereinheit aus den ein oder mehreren Parametern der Stimulationsereignisse (2) und den ein oder mehreren Parametern der elektrischen Aktivierungen (8) im Zeitrahmen (6) ableitet, ob der Teil des menschlichen Herzens im Zeitrahmen (6) erfolgreich auf den Stimulus synchronisiert wurde,
wobei das Steuersystem (1) eine Anzeige (10) umfasst,
wobei die Steuereinheit bestimmt, ob die Synchronisation in Echtzeit während des Zeitrahmens (6) erfolgreich war, wobei die Steuereinheit die Bestimmung, ob die Synchronisation erfolgreich war, an die Anzeige (10) des Steuersystems (1) sendet, um die Bestimmung einem Benutzer anzuzeigen.

## Revendications

1. Système de commande pour détecter un entraînement réussi d'une partie d'un cœur humain, en particulier d'une oreillette,
le système de commande (1) générant des événements de stimulation (2) par l'application d'énergie électrique à des électrodes de stimulation (3) placées au niveau de la partie du cœur humain pour entraîner la partie du cœur humain et générant des données de stimulation relatives aux événements de stimulation (2), les données de stimulation comprenant une fenêtre temporelle (6) dans laquelle les événements de stimulation (2) se produisent, le système de commande (1) mesurant des tensions électriques au niveau d'électrodes de mesure (3) placées au niveau de la partie du cœur humain et générant des données d'électrogramme intracardiaque (7) à partir des mesures, les données d'électrogramme intracardiaque (7) comprenant plusieurs activations électriques (8) de la partie du cœur humain dans la fenêtre temporelle (6),
le système de commande (1) détectant au moins certaines des activations électriques (8) dans la fenêtre temporelle (6),
le système de commande (1) dérivant un ou plusieurs paramètres des événements de stimulation (2) à partir des données de stimulation, le système de commande (1) dérivant un ou plusieurs paramètres des activations électriques (8) dans la fenêtre temporelle (6), le système de commande (1) dérivant, à partir du ou des paramètres des événements de stimulation (2) et du ou des paramètres des activations électriques (8) dans la fenêtre temporelle (6), si la partie du cœur humain a été entraînée avec succès dans la fenêtre temporelle (6),
le système de commande (1) comprenant un dispositif d'affichage (10),
le système de commande (1) déterminant si l'entraînement a réussi en temps réel pendant la fenêtre temporelle (6), le système de commande (1) envoyant la détermination du fait que l'entraînement a réussi au dispositif d'affichage (10) du système de commande (1) pour afficher le résultat de la détermination à un utilisateur,
le système de commande (1), après avoir déterminé que l'entraînement a réussi, arrêtant de générer des événements de stimulation (2), ou, un utilisateur pouvant entrer un signal d'arrêt et mettre fin à la stimulation.

2. Système de commande selon la revendication 1, **caractérisé en ce que** les données de stimulation comprennent un temps d'un début de la fenêtre temporelle (6), **en ce que** les données d'électrogramme intracardiaque (7) comprennent plusieurs activations électriques (8) de la partie du cœur humain avant la fenêtre temporelle (6), **en ce que** le système de commande (1) détecte au moins certaines des activations électriques (8) avant la fenêtre temporelle (6), **en ce que** le système de commande (1) dérive un ou plusieurs paramètres des activations électriques (8) avant la fenêtre temporelle (6), de préférence, **en ce que** le ou les paramètres des activations électriques (8) avant la fenêtre temporelle (6) comprennent des intervalles d'activation.

3. Système de commande selon la revendication 1 ou 2, **caractérisé en ce que** le ou les paramètres des événements de stimulation (2) comprennent un temps des événements de stimulation (2), **en ce que** le ou les paramètres des activations électriques (8) dans la fenêtre temporelle (6) comprennent un temps des activations électriques (8), **en ce que** le système de commande (1) dérive des différences temporelles entre les événements de stimulation (2) et des activations électriques (8) ultérieures dans la fenêtre temporelle (6) pour plusieurs événements de stimulation (2) pour dériver si l'entraînement a réussi dans la fenêtre temporelle (6), de préférence, **en ce que** le système de commande (1) dérive un paramètre statistique, en particulier un écart type, à partir des différences temporelles et si le paramètre statistique satisfait à un critère prédéfini, conclut que l'entraînement a réussi, plus préférablement, **en ce que** le critère prédéfini est basé sur le ou les paramètres des événements de stimulation (2) et/ou sur le ou les paramètres des événements d'activation avant la fenêtre temporelle (6).

4. Système de commande selon l'une des revendications précédentes, **caractérisé en ce que** le système de commande (1) affiche les différences temporelles entre les événements de stimulation (2) et les activations électriques (8) ultérieures.

5. Système de commande selon l'une des revendications précédentes, **caractérisé en ce que** le ou les paramètres des événements de stimulation (2) comprennent un intervalle de stimulation (11), **en ce que** le ou les paramètres des activations électriques (8) dans la fenêtre temporelle (6) comprennent des intervalles d'activation, **en ce que** le système de commande (1) compare l'intervalle de stimulation (11) à plusieurs intervalles d'activation des activations électriques (8) dans la fenêtre temporelle (6) pour dériver si l'entraînement a réussi, de préférence, **en ce que** le système de commande (1) dérive un paramètre statistique, en particulier un écart type, à partir des plusieurs intervalles d'activation des activations électriques (8) dans la fenêtre temporelle (6) par rapport à l'intervalle de stimulation (11) et si le paramètre statistique satisfait à un critère prédéfini, conclut que l'entraînement a réussi, plus préférablement, **en ce que** le critère prédéfini est basé sur le ou les paramètres des événements de stimulation (2).

6. Système de commande selon l'une des revendications précédentes, **caractérisé en ce que** les données d'électrogramme intracardiaque (7) comprennent au moins une activation électrique (8) de la partie du cœur humain après la fenêtre temporelle (6), **en ce que** le système de commande (1) après avoir déterminé que l'entraînement a réussi détecte la première activation électrique (8) après la fenêtre temporelle (6) et dérive un intervalle de post-stimulation (12) à partir de la première activation électrique (8) après la fenêtre temporelle (6) et d'une dernière activation électrique (8) dans la fenêtre temporelle (6).

7. Système de commande selon l'une des revendications 2 à 6, **caractérisé en ce que** le système de commande (1) dérive un modèle de morphologie avant la fenêtre temporelle (6) à partir d'au moins certaines des activations électriques (8) avant la fenêtre temporelle (6), et, **en ce que** le système de commande (1) utilise le modèle de morphologie pour détecter des activations électriques (8) dans la fenêtre temporelle (6) et/ou pour différencier entre des activations électriques (8) et des artefacts de stimulation dans la fenêtre temporelle (6) et/ou pour déterminer si l'entraînement a réussi et/ou pour afficher et/ou mettre en évidence un canal (9) de l'électrogramme intracardiaque.

8. Système de commande pour détecter un entraînement réussi selon l'une des revendications précédentes et pour afficher des informations d'entraînement,
le système de commande (1) générant des événements de stimulation (2) par l'application d'énergie électrique à des électrodes de stimulation (3) placées au niveau de la partie du cœur humain pour entraîner la partie du cœur humain et générant des données de stimulation relatives aux événements de stimulation (2), les données de stimulation comprenant une fenêtre temporelle (6) dans laquelle les événements de stimulation (2) se produisent et un intervalle de stimulation (11), le système de commande (1) mesurant des tensions électriques au niveau d'électrodes de mesure (3) placées au niveau de la partie du cœur humain et générant des données d'électrogramme intracardiaque (7) à partir des mesures, les données d'électrogramme intracardiaque (7) comprenant plusieurs activations électriques (8) de la partie du cœur humain dans la fenêtre temporelle (6),
le système de commande (1) comprenant un dispositif d'affichage (10),
le système de commande (1) affichant pendant la fenêtre temporelle (6) des sections (13) de l'électrogramme intracardiaque de manière empilée.

9. Système de commande selon la revendication 8, **caractérisé en ce que** le système de commande (1) affiche les sections (13) de telle sorte que des sections (13) ultérieures sont affichées l'une au-dessus ou en dessous de l'autre tandis que la dimension temporelle de chaque section (13) est orientée latéralement.

10. Système de commande selon la revendication 8 ou 9, **caractérisé en ce que** le système de commande (1) détermine une corrélation entre au moins certaines des sections (13) et dérive un indicateur de qualité, en particulier pour la détermination du succès de l'entraînement, à partir de la corrélation.

11. Système de commande selon l'une des revendications 8 à 10, **caractérisé en ce que** l'utilisateur peut déplacer un début des sections (13) par l'intermédiaire d'une entrée utilisateur.

12. Unité de commande pour détecter un entraînement réussi d'une partie d'un cœur humain, en particulier d'une oreillette, adaptée pour faire partie d'un système de commande (1) selon l'une des revendications précédentes, l'unité de commande recevant des données de stimulation, les données de stimulation comprenant une fenêtre temporelle (6) dans laquelle les événements de stimulation (2) se produisent,
l'unité de commande recevant ou générant des données d'électrogramme intracardiaque (7), les données d'électrogramme intracardiaque (7) comprenant plusieurs activations électriques (8) de la partie du cœur humain dans la fenêtre temporelle (6),
l'unité de commande détectant au moins certaines des activations électriques (8) dans la fenêtre temporelle (6),
l'unité de commande dérivant un ou plusieurs paramètres des événements de stimulation (2) à partir des données de stimulation, l'unité de commande dérivant un ou plusieurs paramètres des activations électriques (8) dans la fenêtre temporelle (6),
l'unité de commande dérivant, à partir du ou des paramètres des événements de stimulation (2) et du ou des paramètres des activations électriques (8) dans la fenêtre temporelle (6), si la partie du cœur humain a été entraînée avec succès dans la fenêtre temporelle (6),
le système de commande (1) comprenant un dispositif d'affichage (10),
l'unité de commande déterminant si l'entraînement a réussi en temps réel pendant la fenêtre temporelle (6), l'unité de commande envoyant la détermination du fait que l'entraînement a réussi au dispositif d'affichage (10) du système de commande (1) pour afficher le résultat de la détermination à un utilisateur.
